# EUROPEAN PATENT APPLICATION

(11) **EP 3 910 336 A1**
(43) Date of publication of application: **17.11.2021**
(21) Application number: 20174351.5
(22) Date of filing: 13.05.2020
(51) Int. Cl.: G01N 33/543, G01N 27/327

(54) **LIFETIME PREDICTION/DETECTION OF BIOMARKER SENSOR**

(71) Applicant: Koninklijke Philips N.V., 5656 AG Eindhoven (NL)
(72) Inventor: HUIJBREGTS, Laurentia Johanna, 5656 AE Eindhoven (NL); VAN LIESHOUT, Ron Martinus Laurentius, 5656 AE Eindhoven (NL); JOHNSON, Mark Thomas, 5656 AE Eindhoven (NL); GERHARDT, Lutz Christian, 5656 AE Eindhoven (NL); PELSSERS, Eduard Gerard Marie, 5656 AE Eindhoven (NL)
(74) Representative: Philips Intellectual Property & Standards

(57) **Abstract**

The present invention relates to biofluid monitoring. It is proposed to use a system and a method to detect when a biomarker sensor with a regenerable surface has degraded and/or predicting when the sensor will degrade. At least one of the following methods are used for the detection: counting the number of times the surface has been regenerated, determining the cumulative amount of biomolecules measured with the device, detecting an increased/decreased voltage change or pH change needed to release the biomarkers from the capture surface, detecting deviations of the sensor raw signal signatures from factory calibration signals, and using tracer analyte molecules comprising analyte molecules bound to magnetic beads.

## Description

### FIELD OF THE INVENTION

The present invention relates to lifetime prediction or lifetime detection of an analyte sensor.

### BACKGROUND OF THE INVENTION

Biofluids, such as blood, interstitial fluid, sweat, urine and saliva, contain analytes, the concentration of which is informative of the person's health. Examples of such analytes, which are also referred to as biomarkers, are cholesterol, lactate, cortisol and glucose, etc. Continuous or semi-continuous monitoring of biomarkers can help for diagnosis or effective treatment. A sensor that is capable of measuring one or more biomarkers could be put on or in the body or in a biofluid stream to enable continuous or semi-continuous monitoring. To fit in a clinical workflow, such sensors should after application on/in the patient by clinical staff preferably last for multiple days, without additional handling required from a nurse.

When monitoring biomarkers, it may be important that the measurement is accurate. In order to make the right diagnosis or to treat or intervene at appropriate moments, the clinical staff needs to be able to trust the measurements. They should therefore be informed when the accuracy of the sensor is low or alternatively be able to adapt the calibration to the age/status of the sensor. Preferably, they are already warned beforehand such that they can replace the sensor by a new one before the old one becomes useless.

### SUMMARY OF THE INVENTION

There may be a need to improve the reliability of biofluid monitoring.

The present invention is defined by the independent claims, wherein further embodiments are incorporated in the dependent claims. It should be noted that the following described aspects of the invention apply also for the system, the method, and the computer program element.

According to a first aspect of the present invention, there is provided a system for detecting an analyte in a fluid sample of a subject. The system comprises an analyte sensor and a measurement unit. The analyte sensor comprises an analyte sensor matrix having a capture surface with receptors being immobilized thereon for reversibly binding analyte molecules in the fluid sample and a regeneration assembly configured to regenerate the capture surface such that the bound analyte molecules are released from the capture surface. The measurement unit is configured to perform measurements to provide a measured value correlated with a quality of the capture surface. The measured value allows for determining when the analyte sensor matrix has degraded and/or for predicting when the analyte sensor matrix will degrade.

Accordingly, a system is proposed for detecting when an analyte sensor with a regenerable capture surface has degraded and/or for predicting when the sensor will degrade. Based on this information, necessary actions, such as replacement of the analyte sensor, or recalibration of the analyte sensor, may be implemented to prevent inaccurate measurements due to degradation or failure of the analyte sensor. This may allow the clinical staff to make the right diagnosis or to treat or intervene at appropriate moments.

The system comprises an analyte sensor with a regenerable capture surface with receptors being immobilized thereon. In some examples, the analyte sensor may detect body fluids (e.g. sweat, sebum and interstitial fluid) derived from the skin of a subject, i.e. person/animal of which the biofluid is used, and may be associated with the continuous or semi-continuous monitoring of the subject, in particular, the fluid of a subject and/or properties of the fluid of the subject. In some examples, the analyte sensor may detect other body fluids, such as blood, urine, and saliva. The analyte sensor may be, for example, a wearable patch on the skin that measures sweat, an implantable device in interstitial fluid or blood, a toothbrush that measures saliva, a baby bottle that measures milk, a breast pump that measures milk, a toilet sensor that measures urine, a blood measurement outside the body, etc.

The receptor may refer to any detector molecule, either synthetic or natural in origin, which has reasonable affinity and specificity for one or more analyte molecules. The interaction between the receptors and the analyte molecules produces an effect measurable by a transducer, which outputs a measurable signal proportional to the presence of the target analyte in the fluid sample. The immobilized receptors and analyte molecules may also be referred to as biological binding partners, which may include the variety of known ligands. For example, one skilled in the art will appreciate that the biological binding partners may include e.g. an antigen or antibody, a hormone or neurotransmitter and a receptor, a substrate or allosteric effector and an enzyme, lectins and sugars, DNA or RNA structures, such as aptamers and their binding species (including other DNA or RNA species or binding protein), proteins, biotin and adivin or streptavidin systems, enzymes and their substrates and inhibitors, lipid binding systems, and combinations thereof. The binding of the analyte molecules and the receptors forms ligand-receptor complexes.

The system further comprises a measurement unit (e.g. a processor) configured to provide a measured value, which is indicative of a degradation of the regenerable capture surface, thereby allowing the estimation of the degradation, which the analyte sensor has undergone in the past or will undergo in the future. The measured value is correlated with a quality of the regenerable capture surface. In an example, the quality of the capture surface is high if all or almost all receptors on the regenerable capture surface specifically bind to the biomolecule of interest and not to other molecules in the fluid sample. In another example, the quality of the capture surface is high if all or almost all receptors on the regenerable capture surface have a high affinity to the biomolecule of interest (i.e. easily captures the molecule). In a further example, the quality of the capture surface is high if all or almost all receptors on the regenerable capture surface bind a certain amount of biomolecules of interest in a certain amount of time (i.e. binding speed).

In the following, some examples of the measured value are provided:
- In an example, the measurement unit may provide a measured value by counting the number of times the regenerable capture surface has been regenerated.
- In another example, the measurement unit may provide a measured value by determining a cumulative amount of biomolecules of interest measured with the analyte sensor.
- In a further example, the measurement unit may provide a measured value by detecting a characteristic of the regeneration and release process, such as an increased/decreased voltage change, current change, or pH change needed to release the biomarkers (e.g. antigens) from the regenerable capture surface with receptors (e.g. antibodies).
- In a further example, the measurement unit may provide a measured value by detecting an optical out-of-calibration signal of an optical sensor to measure ligand-receptor (e.g. Ab-Ag) binding. Examples of the optical out-of-calibration signal may include, but are not limited to, permanent drift or offset of optical signal (e.g. light intensity, absorption, reflection, scattering, etc.) due to deterioration of the regenerable capture surface.
- In a further example, the measurement unit may provide a measured value by detecting an electrical or mechanical out-of-calibration signal due to deterioration of the regenerable capture surface. Examples of the out-of-calibration signal may include, but are not limited to, permanent drift or offset of resonance frequency in a mechanical resonance sensor (e.g. quartz crystal microbalance sensor) for the detection of ligand-receptor (e.g. Ab-Ag) binding.
- In a further example, the measurement unit may provide a measured value by using tracer analyte molecules in form of analyte molecules bound to magnetic beads.

The measurement unit may further calculate a reliability measure based on the measured value. The reliability measure may be, for example, the accuracy/uncertainty of the biomarker concentration, a quality index (e.g. a number between 1 and 10, or a binary defect indication, i.e. whether or not the regenerable capture surface is defect), or the time to become inaccurate at current usage.

According to an embodiment of the present invention, the system further comprises a processing unit configured to determine when the analyte sensor matrix has degraded and/or to predict when the analyte sensor matrix will degrade based on the measured value.

Accordingly, inaccurate measurements due to degradation or failure of the analyte sensor may be avoided based on the detection or prediction results.

According to an embodiment of the present invention, the system further comprises a calibration assembly configured to calibrate the analyte sensor using a set of calibration parameters, which is adaptable to the measured value.

As regeneration is triggered many times, the receptors might lose their structure and thereby their ability to capture analyte molecules and/or the amount of active receptors on the regenerable capture surface may be reduced. By adapting the sensor calibration as a consequence of the quality of the regenerable capture surface, the accuracy of the analyte sensor may be increased.

According to an embodiment of the present invention, the measurement unit is configured to count a number of times the capture surface has been regenerated.

In other words, it is proposed to assess the quality of the regenerable capture surface by counting the times that the regenerable capture surface or a certain part of the regenerable surface is regenerated. For example, a microcontroller may be used to count the regeneration events, e.g. active voltage sweeps, upon first time usage.

According to an embodiment of the present invention, the measurement unit is configured to determine a cumulative amount of analyte molecules measured with the analyte sensor matrix.

In other words, it is proposed to determine the actual cumulative analyte molecule concentration captured over time, because this is proportional to how many ligand-receptor complexes have been formed and released again. The cumulative amount of analyte molecules may provide a more accurate quality assessment of the sensor.

According to an embodiment of the present invention, the measurement unit is configured to determine at least one of the following measured values: total time of a presence of the receptors in a harsh environment, replenishment of fluid, and
amount of hydrogen ions in the fluid sample.

The degradation of the regenerable capture surface is linked to the total time the receptors are in a harsh environment (e.g. low or high pH), the replenishment of fluid (i.e. flow-rate) and the buffer capacity of the fluid (i.e. in which matrix is measured) and/or a combination thereof. The inclusion of one or more of these measurement values may improve the quality assessment of the regenerable capture surface.

According to an embodiment of the present invention, the measurement unit is configured to measure at least one of a voltage change, a current change, or a change of hydrogen ions amount needed to release the bound analyte molecules from the capture surface.

In other words, it is proposed to perform a direct measure of the quality of the capture surface. In some examples, such a direct measure may be an increased or a decreased electrical characteristic (e.g. voltage or current change) needed to release the analyte molecules from the capture surface. In some examples, such a direct measure may be an increased or decreased pH change needed to release the analyte molecules from the capture surface.

This will be explained hereafter and in particular with respect to example 2a in the detailed description of embodiments.

According to an embodiment of the present invention, the system comprises a detector configured to detect an amount of analyte molecules bound to the receptors and to generate a detection signal correlated with the amount of the bound analyte molecules.
The measurement unit is configured to determine whether the detection signal is within a range of signal intensity that defines an operating window of the analyte sensor and to generate an out-of-calibration signal indicative of a deterioration of the capture surface when the detection signal is outside the operating window.

In other words, it is proposed to perform the end-of-lifetime prediction or detection by comparing, e.g. continuously or semi-continuously comparing, sensor raw signal signatures with factory or first-time use calibration signals. Any significant deviation, such as permanent offset or drift of sensor raw signal or signal exceeding an upper or lower threshold value, may be used as wear-out indicator.

This will be explained hereafter and in particular with respect to example 2b in the detailed description of the embodiments.

According to an embodiment of the present invention, the processing unit is configured to determine whether the detection signal is within the operating window after a regeneration.

In other words, it is possible to look at the signal, e.g. optical, electrical, or mechanical signal, only directly after regenerations. In this example, there are no ligand-receptor complexes, but only unbounded analyte molecules present at the capture surface. Accordingly, the signals can directly be compared to each other without having to take into account fluctuations by the variable presence of ligand-receptor bonds.

According to an embodiment of the present invention, the detector comprises at least one of:
- an optical sensor configured to generate an optical signal, which is correlated with the amount of the bound analyte molecules;
- an electrochemical sensor configured to transform electrochemical information into an analytically useful signal, which is correlated with an amount of the bound analyte molecules;

- a magnetic sensor configured to employ magnetic particles and/or crystals for detecting biological interactions by measuring changes in magnetic properties or magnetically induced effects, which are correlated with an amount of the bound analyte molecules; and
- a mechanical resonance sensor configured to generate an electrical signal indicative of a change of mechanical resonance frequency of the mechanical resonance sensor, which is correlated with the amount of the bound analyte molecules.

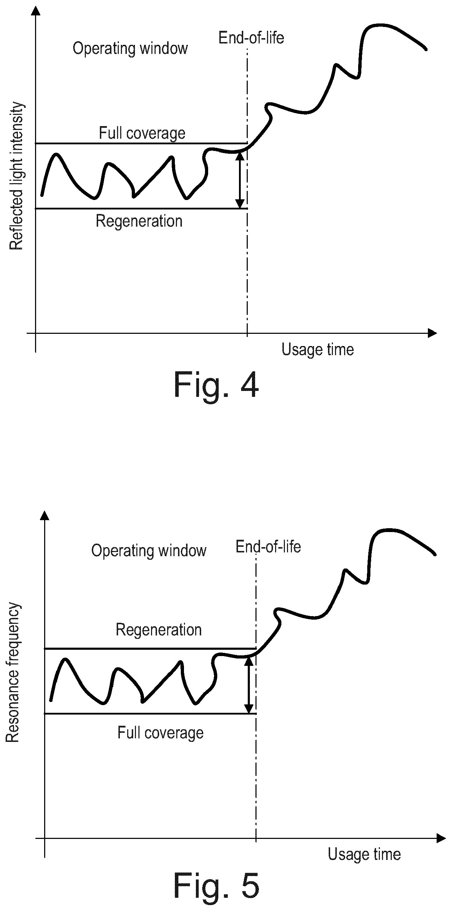

This will be explained hereafter and in particular with respect to the examples illustrated in Figs. 4 and 5.

According to an embodiment of the present invention, the operating window is derived from factory calibration signals or first-time use calibration signals.

According to an embodiment of the present invention, the system comprises a fluid channel arranged such that the fluid sample flows through the fluid channel, wherein the fluid channel has a first channel surface with a collection surface arranged thereon and a second channel surface with the capture surface arranged opposite to the collection surface. The system further comprises a plurality of analyte molecules as tracer analyte molecules, each tracer analyte molecule being attached to a respective magnetic bead. The system further comprises a magnet arrangement, which comprises a first electromagnetic magnet configured to be powered to attract the plurality of tracer analyte molecules to the collection surface and a second electromagnetic magnet configured to be powered to attract the plurality of tracer analyte molecules to the capture surface. The system further comprises a power supply configured to supply power to the magnet arrangement. The system further comprises a controller configured to control the power supply to depower the first electromagnetic magnet and the second electromagnetic magnet to release the tracer analyte molecules from the collection surface to allow for binding of the plurality of tracer analyte molecules to the receptors on the capture surface after an incubation time, and to power, after the incubation time, the first electromagnetic magnet to apply a magnetic field of a defined field strength. The system further comprises a detector configured to detect an amount of tracer analyte molecules on one of the capture surface and the collection surface. The measurement unit is configured to correlate the detected amount of tracer analyte molecules on one of the capture surface and the collection surface with the quality of the capture surface.

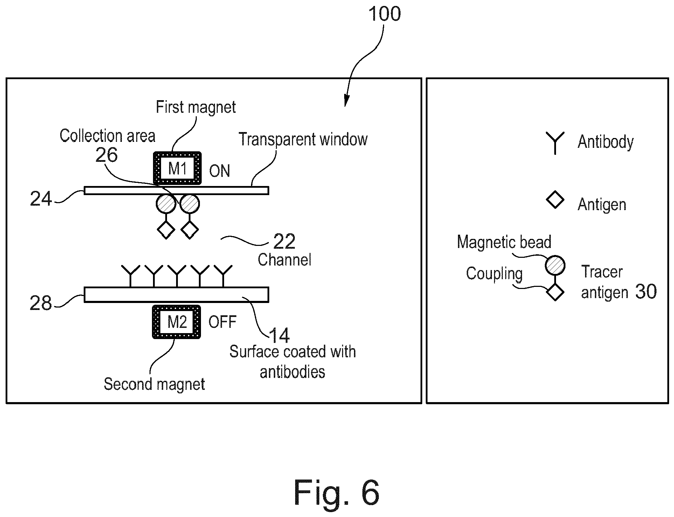

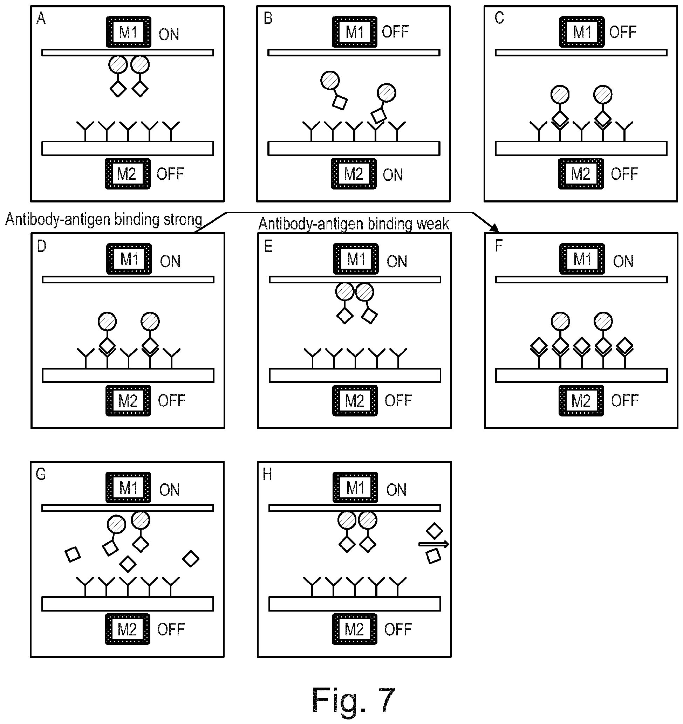

This will be explained hereafter and in particular with respect to the example illustrated in Figs. 6 and 7A to 7H.

According to an embodiment of the present invention, the system further comprises a user interface configured to notify a user of the measured value.

In an example, the user interface is a display for displaying the measured value.

In a further example, the user interface is configured to send a notification or to provide an alarm to a user, such as a person whose biofluid is used or a caregiver (e.g. nurse).

According to a second aspect of the present invention, there is provided a method for lifetime prediction or lifetime detection of an analyte sensor. The method comprises the following steps:
- providing an analyte sensor that comprises:
   - an analyte sensor matrix having a capture surface with receptors being immobilized thereon for reversibly binding analyte molecules in the fluid sample; and
   - a regeneration assembly configured to regenerate the capture surface such that the bound analyte molecules are released from the capture surface; and
- performing measurements, by a measurement unit, to provide a measured value correlated with a quality of the capture surface, wherein the measured value allows for determining when the analyte sensor matrix has degraded and/or for predicting when the analyte sensor matrix will degrade.

According to a third aspect of the present invention, there is provided a program element for lifetime prediction or lifetime detection of an analyte sensor, which program element, when being executed by a processor, is adapted to carry out the method according to the second aspect and any associated example.

Advantageously, the benefits provided by any of the above aspects equally apply to all of the other aspects and vice versa.

As used herein, the term "magnetic beads" refer to beads, which are magnetically responsive. It is noted that the magnetic beads are not permanent magnetic beads, as they can aggregate due to magnetic attraction by themselves. Rather, the magnetic beads are not magnetic without external magnetic field. The magnetic beads may include paramagnetic or super-paramagnetic particles, and/or crystals. For magnetic beads, the magnetically responsive material may constitute substantially all of a bead or one component only of a bead. The remainder of the beads may include, among other things, polymeric material, coatings, and moieties, which permit attachment of an assay reagent. The magnetic beads may be any of a wide variety of shapes, such as spherical, generally spherical, egg shaped, disc shaped, cubical and other three-dimensional shapes. The magnetic beads may range in size from nanometers to microns in diameter.

The term "controller" is used generally to describe various apparatus relating to the operation of an apparatus, system, or method. A controller can be implemented in numerous ways (e.g., such as with dedicated hardware) to perform various functions discussed herein. A "processor" is one example of a controller that employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects of the present disclosure discussed herein. The terms "program" or "computer program" are used herein in a generic sense to refer to any type of computer code (e.g., software or microcode) that can be employed to program one or more processors or controllers.

The term "user interface" as used herein refers to an interface between a human user or operator and one or more devices that enables communication between the user and the device(s). Examples of user interfaces that may be employed in various implementations of the present disclosure include, but are not limited to, switches, potentiometers, buttons, dials, sliders, track balls, display screens, various types of graphical user interfaces (GUIs), touch screens, microphones and other types of sensors that may receive some form of human-generated stimulus and generate a signal in response thereto.

The term "unit" as used herein may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 schematically shows an example of a system for lifetime prediction or lifetime detection of an analyte sensor.
Fig. 2 shows an example of voltage change decrease.
Fig. 3A schematically illustrates an example of a fully regenerated capture surface after the factory or first-time use calibration.
Fig. 3B illustrates the fully functional capture surface of Fig. 3A with full coverage of Ab-Ag binding complexes.
Fig. 3C illustrates the irreversibly deteriorated capture surface of Fig. 3A
Fig. 4 illustrates the drifting away of optical signal as a lifetime indicator.
Fig. 5 illustrates the drifting away of the resonance frequency as a lifetime indicator.
Fig. 6 schematically illustrate a further example of the system that uses tracer analyte molecules comprising analyte molecules bound to magnetic beads.
Figs. 7A to 7H schematically illustrate the procedure to check the quality of the capture surface.
Fig. 8 illustrates a flowchart of a method for lifetime prediction or lifetime detection of an analyte sensor.

### DETAILED DESCRIPTION OF EMBODIMENTS

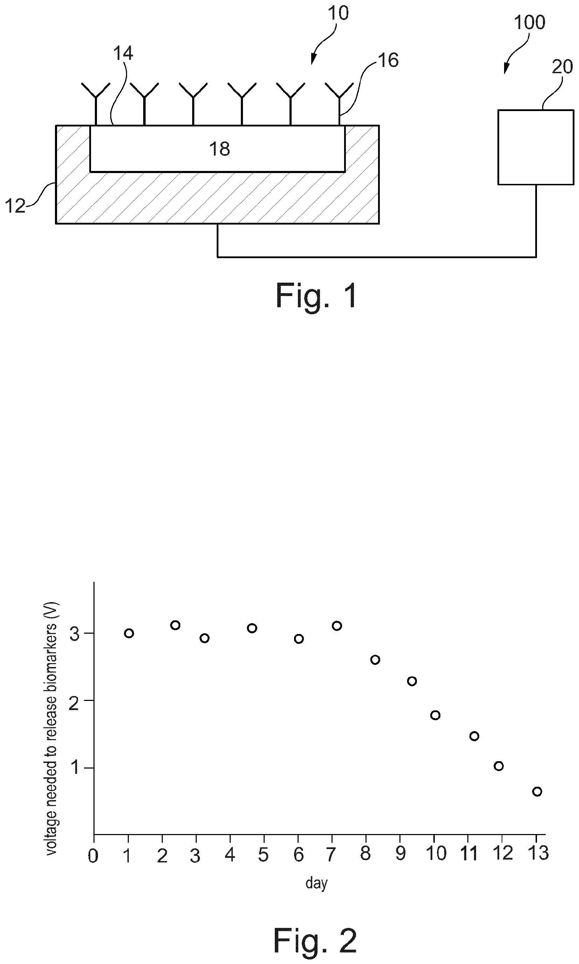

Fig. 1 schematically shows a system 100 for detecting an analyte in a fluid sample of a subject. The system 100 comprises an analyte sensor 10, such as a wearable patch on the skin that measures sweat, an implantable device in interstitial fluid or blood, a toothbrush that measures saliva, a baby bottle that measures milk, a breast pump that measures milk, a toilet sensor that measures urine, a blood measurement outside the body, etc.

The analyte sensor 10 comprises an analyte sensor matrix 12 having a capture surface 14 with receptors 16 being immobilized thereon for reversibly binding analyte molecules in the fluid sample.

The analyte sensor 10 further comprises a regeneration assembly 18 configured to regenerate the capture surface 14 such that the bound analyte molecules are released from the capture surface 14. To remove the analyte from the capture surface, three types of elution are possible. In an example, the regeneration assembly 18 may be configured to treat the capture surface with harsh conditions (i.e. pH) to break the interaction between the immobilized receptors and the analyte molecules. For example, the preferred pH for a specific Ag-Ab complex denaturation is in the acidic range around pH 2-3. In another example, the regeneration assembly may be configured to add a saturating amount of a small compound that mimics the binding site. In a further example, the regeneration assembly may be configured to treat with an agent that induces an allosteric change that releases the analyte molecules.

The system 100 further comprises a measurement unit 20 configured to perform measurements to provide a measured value correlated with a quality of the capture surface. The measured value allows for determining when the analyte sensor matrix has degraded and/or for predicting when the analyte sensor matrix will degrade.

Thus, the measurement unit 20 may be part of, or include an ASIC, an electronic circuit, a processor (shared, dedicated, or group) and/or memory (shared, dedicated, or group) that execute one or more software or firmware programs, a combinational logical circuit, and/or other suitable components that provide the described functionality.

Optionally, the measurement unit may be configured to further calculate a reliability measure based on the measured value. The reliability measure is, for example, the accuracy/uncertainty of the biomarker concentration, a quality index (e.g. a number between 1 and 10, or a binary defect indication, i.e. whether or not the regenerable capture surface is defect), or the time to become inaccurate at current usage.

Optionally, the measured value and/or the reliability measure may be used as follows:
- It is shown on the screen together with the value of the biomarker concentration.
- A notification is sent to (e.g. the mobile phone of) the user (e.g. a patient or a caregiver, e.g. nurse, or an alarm will sound when the reliability measure has become low.
- Measured biomarker concentrations are not taken into account for further analysis (e.g. to calculate an early warning score) when the accompanying reliability measure is low.
- An alternative calibration is used adjusted to the actual quality of the sensor.

Optionally, the system 100 may further comprise a processing unit (not shown) configured to determine when the analyte sensor matrix has degraded and/or to predict when the analyte sensor matrix will degrade based on the measured value. Accordingly, inaccurate measurements due to degradation or failure of the analyte sensor may be avoided based on the detection or prediction results. This will be explained hereafter and in particular with reference to Fig. 2.

Thus, the processing unit may be part of, or include an ASIC, an electronic circuit, a processor (shared, dedicated, or group) and/or memory (shared, dedicated, or group) that execute one or more software or firmware programs, a combinational logical circuit, and/or other suitable components that provide the described functionality. Furthermore, such processing unit may be connected to volatile or non-volatile storage, display interfaces, communication interfaces and the like as known to a person skilled in the art.

Optionally, the system 100 may further comprise a calibration assembly (not shown) configured to calibrate the analyte sensor using a set of calibration parameters, which is adaptable to the measured value. As regeneration is triggered many times, the receptors might lose their structure and thereby their ability to capture analyte molecules and/or the amount of active receptors on the regenerable capture surface may be reduced. By adapting the sensor calibration as a consequence of the quality of the regenerable capture surface, the accuracy of the analyte sensor may be increased.

Optionally, the system 100 may further comprise a user interface (not shown) configured to notify a user of the measured value. For example, the user interface may be a display for displaying the measured value. In a further example, the user interface is configured to send a notification or to provide an alarm to a user, such as a caregiver (e.g. nurse).

In the following, some examples of the measured value are described that is correlated with the quality of the capture surface, including indirect measures of the quality of the surface described in section "Example 1" below and direct measures of the quality of the surface described in section "Example 2" below.

### Example 1: Quality changes as a function of usage

The more often an analyte binds to the immobilized receptor and is released again (e.g. by applying a voltage or pH change), the more probable it becomes that defects start to occur in the immobilized receptors. The immobilized receptors may then become less specific to the analyte molecules (i.e. the biomarker of interest) and may bind also to other molecules from the biofluid, or the receptors may lose their affinity to the analyte molecules (e.g. although the analyte molecules are present around the empty receptors, no ligand-receptor complex is formed). Both effects decrease the sensitivity and accuracy of the analyte sensor.

### Example 1a: Counting the times that the surface has been regenerated

Therefore, in a first example, it is proposed to assess the quality of the capture surface by counting the times that the surface or a certain part of the surface has been regenerated. In this example, the measurement unit may be a microcontroller that counts the regeneration events, e.g. active voltage sweeps upon first time usage. This example gives a measure for the quality of the surface, which can further be utilized to improve operation of the device.

### Example 1b: Determining the cumulative amount of biomolecules measured with the device

However, regeneration may not always take place only when the sensor is completely exhausted. Therefore, in a second example, it is proposed to determine the actual cumulative biomarker concentration captured over time, because this is proportional to how many ligand-receptor complexes have been formed and released again. This will provide a more accurate quality assessment of the sensor.

Further, since the degradation of the immobilized receptors is linked to the total time the immobilized receptors are in a harsh environment (i.e. low or high pH), the replenishment of fluid (i.e. flow-rate), and the buffer capacity of the biofluid (i.e. in which matrix is measured), and/or a combination thereof, the inclusion of one or more of the above-described measured values may improve the quality assessment of the capture surface.

### Example 2: Dynamic assessment of quality change

The above-described examples propose to use the number of times a surface has been regenerated and the cumulative biomarker concentration are as an assessment of quality. However, these are in fact indirect measures of the quality of the surface and do not take into account the details (e.g. environmental aspects like temperature, pH etc.) during the regeneration process. As a consequence, it might be that more receptors than expected are still intact, even if the surface has been regenerated many times, or, the other way around, that less receptors than expected are intact after regeneration only a couple of times.

Therefore, in the following examples, a direct measure of the quality of the capture surface is proposed.

### Example 2a: Detection of a characteristic of the regeneration/release process (e.g. increased/decreased voltage, current, and/or pH change)

In some examples, such direct measures may be an increased or a decreased electrical characteristic, such as voltage or current change or indeed in the pH change, needed to release the biomarkers from the capture surface. Fig. 2 shows an example of voltage change decrease. The dots represent the occasions at which the surface was regenerated. In this example, until Day 7 the required voltage is around 3 V. From Day 8 onwards the voltage starts to decrease, which is a sign of degradation. It holds mutatis mutandis for a voltage or current change increase or a pH decrease/increase, as an increase/decrease in voltage change or pH change needed to release the antigens indicates that the non-covalent bonds between the ligands and receptors (e.g. antigens and antibodies) are harder or easier to break than before and thus that the receptors (e.g. antibodies) has changed its structure.

When the system normally (i.e. outside regeneration) functions at 0 V, the voltages given in Fig. 2 are equal to the voltage changes needed for regeneration, whereas when the system normally operates at -1 V, then 1 V should be added to get the voltage change. The system of Fig. 2 may e.g. allow regeneration voltages between 2.5 and 3.5 V. If the required voltage exceeds 3.5 V or is lower than 2.5 V, it might give a warning that the sensor is not reliable anymore.

Instead of or in addition to giving the current quality of the capture surface, predictions are also possible. For example, if it is expected that the end-of-lifetime (i.e. when the analyte sensor becomes insufficiently accurate anymore) is reached when the surface has been regenerated 20 times and when the system measures that the surface has been regenerated once per day already for 10 days, then it could show that, at the current usage, the sensor would last for 10 more days.

### Example 2b: Detection of an optical, mechanical, electrical, and/or magnetic out-of-calibration signal

In some examples, the life-time prediction may be done by comparing, e.g. continuously or semi-continuously comparing, sensor raw signal signatures e.g. for detection of ligand-receptor (e.g. antibody/antigen) binding from factory or first-time use calibration signals. Any significant deviation may be used as wear-out or replacement indicator. The significant deviation may be e.g. permanent offset or drift of sensor raw signal, or signal exceeding an upper or lower threshold value. Alternatively, also the ratio of two signals intensities could be used or differences in two sensor signals.

In a first example, the sensor raw signal may be an optical signal generated by an optical sensor, which is correlated with the amount of the bound analyte molecules. For example, transparent electrodes and optical detectors may be used for label-free detection of regenerated antibodies. In this example, permanent drift or offset of optical signal (e.g. light intensity, absorption, reflection, scattering) due to e.g. mechanical and/or chemical deterioration of regenerable sensor surface may be used for the life-time prediction. Also electrical or electrochemical may be the cause of deterioration due to continuously applied high fields/voltages to regenerate.

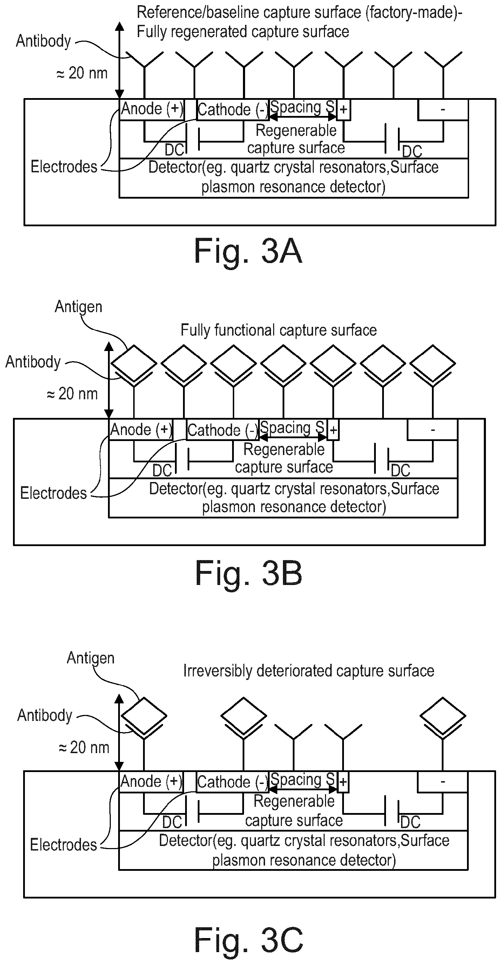

For example, Fig. 3A schematically illustrates an example of a fully regenerated capture surface after the factory or first-time use calibration. Fig. 3B illustrates the fully functional capture surface of Fig. 3A with full coverage of ligand-receptor (e.g. Ab-Ag) binding complexes. Fig. 3C illustrates the irreversibly deteriorated capture surface of Fig. 3A, when the surface has been regenerated many times. The irreversible deterioration of the regenerable sensor surface will cause the permanent drift or offset of optical signal, which may be used for the life-time prediction.

For example, the drifting away of optical signal can be used as a lifetime indicator. For example, as illustrated in Fig. 4, the detection signal may normally alternate around a certain signal intensity between two values representing complete ligand-receptor binding and full regeneration. Alternatively, one can look at the optical signal only directly after regenerations (i.e. there are no ligand-receptor complexes, but only unbounded receptors present at the capture surface) so that the optical signals can directly be compared to each other without having to take into account fluctuations by the variable presence of ligand-receptor bonds.

In a second example, the sensor raw signal may be an electrical signal generated by a mechanical resonance sensor indicative of a change of mechanical resonance frequency of the mechanical resonance sensor, which is correlated with the amount of the bound analyte molecules and/or antibodies.

For example, it is possible to monitor the change of the resonance frequency of a quartz-crystal microbalance (QCM) / quartz-crystal resonator, which is used as a regenerable capture surface and normally operated in a sensing and activation (i.e. regeneration) mode. Whereas in the sensing mode, the QCM measures the binding kinetics of the ligand-receptor complex; in the latter activation mode, the QCM is used as an active stimulation electrode to induce a pH change. In this example, a third mode, i.e. a wear out detection mode, may be added. Again, a drift or a constant, permanent shift in the resonance frequency may indicate an end-of-life of the analyte sensor. One can expect the resonance frequency also to alternate between two values representing complete ligand-receptor binding and full regeneration. Again, as illustrated in Fig. 5, permanent drift or offset of the resonance frequency in a quartz crystal microbalance sensor for detection of ligand-receptor binding may indicate an end-of-life of the analyte sensor. In some examples, it may be possible to look at the signal at any point in time, while allowing for fluctuations due to more or fewer ligand-receptor complexes. In some other examples, it may be possible to look at only right after regeneration without having to take into account fluctuations by a varying amount of ligand-receptor complexes.

In a third example, the sensor raw signal may be generated by an electrochemical sensor, which is configured to transform electrochemical information into an analytically useful signal, which is correlated with an amount of the bound analyte molecules. In this example, the drifting away of the analytically useful signal may be used as a lifetime indicator.

In a fourth example, the sensor raw signal may be generated by a magnetic sensor, which employs paramagnetic or super-paramagnetic particles, and/or crystals, as a method of detecting biological interactions by measuring changes in magnetic properties or magnetically induced effects, such as changes in coil inductance, resistance or magneto-optical properties. The measured changes in magnetic properties or magnetically induced effects (e.g. changes in coil inductance, resistance or magneto-optical properties) are correlated with an amount of the bound analyte molecules. The particles used in magnetic biosensors may range in size from nanometers to microns in diameter and are coated with a bio-receptor such as an antibody or strand of nucleic acid. Interaction with the target causes physical properties of the particles to change; this might be associated with mobility or size. There are a number of technologies employed to detect the particles in a magnetic biosensor including coils, GMR (giant magnetic resistance) devices, Hall Effect devices and various optical and imaging techniques. Thus, the drifting away of a signal indicative of e.g. changes in coil inductance, resistance or magneto-optical properties may be used as a lifetime indicator.

### Example 2c: Using tracer analyte molecules comprising analyte molecules bound to magnetic beads

Fig. 6 schematically illustrates a further example of the system that uses tracer analyte molecules comprising analyte molecules bound to magnetic beads. Figs. 7A to 7H schematically illustrate the procedure to check the quality of the capture surface. To facilitate understanding of the devices and methods described herein, immunological ligands, i.e. antigens and antibodies, will be described henceforth and the device will be referred to as an immunosensor or a biosensor

As illustrated in Fig. 6, the system 100 may comprise a fluid channel arranged such that the fluid sample flows through the fluid channel 22. The fluid channel 22 has a first channel surface 24 with a collection surface 26 arranged thereon and a second channel surface 28 with the capture surface 14 arranged opposite to the collection surface 26.

The system 100 further comprises a plurality of analyte molecules as tracer analyte molecules 30. Each tracer analyte molecule 30, such as antigens in Fig. 6, is attached to a respective magnetic bead.

The system 100 further comprises a magnet arrangement comprising a first electromagnetic magnet M1 and a second electromagnetic magnet M2. The first electromagnetic magnet M1 is configured to be powered to attract the plurality of tracer analyte molecules 30 to the collection surface 26. The second electromagnetic magnet M2 is configured to be powered to attract the plurality of tracer analyte molecules 30 to the capture surface 14.

The system 100 further comprises a power supply (not shown) configured to supply power to the magnet arrangement.

The system 100 further comprises a controller to control the power supply to carry out the following procedure after a regeneration, which is illustrated in Figs. 7A to 7H.

In Fig. 7A, a small amount of tracer analyte molecules 30 (e.g. tracer antigens) that are hold in place at the collection area 26 by the first electromagnetic magnet M1.

In Fig. 7B, the tracer analyte molecules 30 (e.g. tracer antigens) are released by depowering the first electromagnetic magnet M1 and subsequently by a second electromagnetic magnet M2 attracted towards the receptors (e.g. antibodies) coated on the capture surface 14.

In Fig. 7C, the second electromagnetic magnet M2 is subsequently depowered as well. After an incubation time allowing for binding of these tracer analyte molecules (e.g. tracer antigens) to the receptors (e.g. antibodies).

In Fig. 7D, the first electromagnetic magnet M1 is powered up again applying a magnetic field of defined field strength. When tracer analyte molecules 30 (e.g. tracer antigens) remain bound to the receptors (e.g. antibodies), the receptors (e.g. antibodies) are still in good shape.

In Fig. 7E, when the tracer analyte molecules 30 (e.g. tracer antigens) are pulled off, the receptors (e.g. antibodies) have reached end-of-life. The latter effect can be observed by the collection of these detached tracer analyte molecules (e.g. tracer antigens) at the collection area 26 close to where the first magnetic field is applied. By simply observing these tracer particles one does know that end-of life of the antibodies has been reached. In other words, the measurement unit may correlate the detected amount of tracer analyte molecules on one of the capture surface and the collection surface with the quality of the capture surface.

In an example, the amount of tracer analyte molecules may be detected using an optical sensor. For example, the optical sensor may use the principle of total internal reflection.

In another example, the amount of magnetic beads may be detected to determine the amount of the tracer analyte molecules. Examples of the devices may include, but are not limited to coils, GMR devices, or Hall Effect devices.

In Fig. 7F, a measurement is subsequently carried out by offering a fluid with potential presence of analyte molecules (e.g. antigens). It is noted that the tracer particles are only present in a small amount.

In Fig. 7G, a regeneration protocol is then activated while the first magnetic field is maintained and both the analyte molecules (e.g. antigens) and the tracer analyte molecules (e.g. tracer antigens) are released and by the magnetic field the tracer analyte molecules (e.g. tracer antigens) are pulled towards the collection area 26.

In Fig. 7H, after further washing away the none-tracer analyte molecules, the collected tracer particles are again ready for a new determination of the quality of the antibodies.

One can decide to indeed activate the above-mentioned procedure or one can typically activate the procedure after 5, 10 or maybe after even 50 measurements of analyte molecules (e.g. antigens), depending on an a-priori estimate of the quality of the receptors (e.g. antibodies).

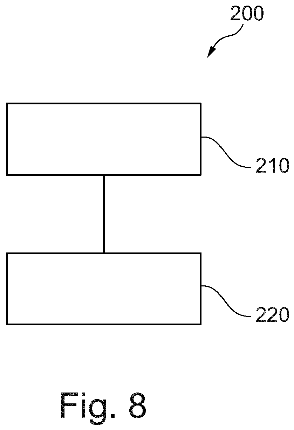

Fig. 8 shows a flowchart of a method 200 for lifetime prediction or lifetime detection of an analyte sensor.

In step 210, an analyte sensor is provided. The analyte sensor comprises an analyte sensor matrix having a capture surface with receptors being immobilized thereon for reversibly binding analyte molecules in the fluid sample, and a regeneration assembly configured to regenerate the capture surface such that the bound analyte molecules are released from the capture surface.

In step 220, a measurement unit performs measurements to provide a measured value correlated with a quality of the capture surface. The measured value allows for determining when the analyte sensor matrix has degraded and/or for predicting when the analyte sensor matrix will degrade.

In an example, the measurement unit may provide a measured value by counting the number of times the regenerable capture surface has been regenerated.

In another example, the measurement unit may provide a measured value by determining a cumulative amount of biomolecules of interest measured with the analyte sensor.

In a further example, the measurement unit may provide a measured value by detecting a characteristic of the regeneration and release process, such as an increased/decreased voltage change or pH change needed to release the biomarkers (e.g. antigens) from the regenerable capture surface with receptors (e.g. antibodies).

In a further example, the measurement unit may provide a measured value by detecting an optical out-of-calibration signal of an optical sensor to measure the ligand-receptor (e.g. Ab-Ag) binding. Examples of the optical out-of-calibration signal may include, but are not limited to, permanent drift or offset of optical signal (e.g. light intensity, absorption, reflection, scattering, etc.) due to deterioration of the regenerable capture surface.

In a further example, the measurement unit may provide a measured value by detecting an electrical or mechanical out-of-calibration signal due to deterioration of the regenerable capture surface. Examples of the out-of-calibration signal may include, but are not limited to, permanent drift or offset of resonance frequency in a mechanical resonance sensor (e.g. quartz crystal microbalance sensor) for detection of e.g. Ab-Ag binding.

In a further example, the measurement unit may provide a measured value by using tracer analyte molecules comprising analyte molecules bound to magnetic beads.

The measurement unit may further calculate a reliability measure based on the measured value. The reliability measure is, for example, the accuracy/uncertainty of the biomarker concentration, a quality index (e.g. a number between 1 and 10, or a binary defect indication, i.e. whether or not the regenerable capture surface is defect), or the time to become inaccurate at current usage.

Optionally, the method may further comprise the step of determining, using a processor, when the analyte sensor matrix has degraded and/or predicting when the analyte sensor matrix will degrade based on the measured value.

Optionally, the method may comprise the step of calibrating, using a calibration assembly, the analyte sensor using a set of calibration parameters, which is adaptable to the measured value.

It should also be understood that, unless clearly indicated to the contrary, in any methods claimed herein that include more than one step or act, the order of the steps or acts of the method is not necessarily limited to the order in which the steps or acts of the method are recited.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. In general, the term "or" as used herein shall only be interpreted as indicating exclusive alternatives (i.e. "one or the other but not both") when preceded by terms of exclusivity, such as "either," or "one of'.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "having," "containing," and the like are to be understood to be open-ended, i.e., to mean including but not limited to.

A person skilled in the art should note that directional terms, such as "above," "below," "upper," "lower," and other like terms are used for the convenience of the reader in reference to the drawings. Also, a person skilled in the art should notice this description may contain other terminology to convey position, orientation, and direction without departing from the principles of the present disclosure.

Furthermore, in this detailed description, a person skilled in the art should note that quantitative qualifying terms such as "generally," "substantially," and other terms are used, in general, to mean that the referred to object, characteristic, or quality constitutes a majority of the subject of the reference. The meaning of any of these terms is dependent upon the context within which it is used, and the meaning may be expressly modified.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A system (100) for detecting an analyte in a fluid sample of a subject, the system comprising:
- an analyte sensor (10), comprising:
- an analyte sensor matrix (12) having a capture surface (14) with receptors (16) being immobilized thereon for reversibly binding analyte molecules in the fluid sample; and
- a regeneration assembly (18) configured to regenerate the capture surface such that the bound analyte molecules are released from the capture surface; and
- a measurement unit (20) configured to perform measurements to provide a measured value correlated with a quality of the capture surface, wherein the measured value allows for determining when the analyte sensor matrix has degraded and/or for predicting when the analyte sensor matrix will degrade.

2. System according to claim 1, further comprising:
- a processing unit configured to determine when the analyte sensor matrix has degraded and/or to predict when the analyte sensor matrix will degrade based on the measured value.

3. System according to claim 1 or 2, further comprising:
- a calibration assembly configured to calibrate the analyte sensor using a set of calibration parameters;
wherein the set of calibration parameters is adaptable to the measured value.

4. System according to any one of the preceding claims,
wherein the measurement unit is configured to count a number of times the capture surface has been regenerated.

5. System according to any one of the preceding claims,
wherein the measurement unit is configured to determine a cumulative amount of analyte molecules measured with the analyte sensor matrix.

6. System according to any one of the preceding claims,
wherein the measurement unit is configured to determine at least one of the following measured values:
- total time of a presence of the receptors in a harsh environment;
- replenishment of fluid; and
- buffer capacity of the fluid sample.

7. System according to any one of the preceding claims,
wherein the measurement unit is configured to measure at least one of a voltage change, a current change, or a pH change needed to release the bound analyte molecules from the capture surface.

8. System according to any one of the preceding claims, further comprising:
- a detector configured to detect an amount of analyte molecules bound to the receptors and generate a detection signal correlated with the amount of the bound analyte molecules;
wherein the measurement unit is configured to determine whether the detection signal is within a range of signal intensity that defines an operating window of the analyte sensor matrix and to generate an out-of-calibration signal indicative of a deterioration of the capture surface when the detection signal is outside the operating window.

9. System according to claim 8,
wherein the processing unit is configured to determine whether the detection signal is within the operating window after a regeneration.

10. System according to claim 8 or 9,
wherein the detector comprises at least one of:
- an optical sensor configured to generate an optical signal, which is correlated with the amount of the bound analyte molecules;
- an electrochemical sensor configured to transform electrochemical information into an analytically useful signal, which is correlated with an amount of the bound analyte molecules;
- a magnetic sensor configured to employ magnetic particles and/or crystals for detecting biological interactions by measuring changes in magnetic properties or magnetically induced effects, which are correlated with an amount of the bound analyte molecules; and
- a mechanical resonance sensor configured to generate an electrical signal indicative of a change of mechanical resonance frequency of the mechanical resonance sensor, which is correlated with the amount of the bound analyte molecules.

11. System according to any one of claims 8 to 10,
wherein the operating window is derived from factory calibration signals or first-time use calibration signals.

12. System according to any one of claims 1 to 3, further comprising:
- a fluid channel (22) arranged such that the fluid sample flows through the fluid channel, wherein the fluid channel has a first channel surface (24) with a collection surface (26) arranged thereon and a second channel surface (28) with the capture surface arranged opposite to the collection surface; and
- a plurality of analyte molecules as tracer analyte molecules (30), each tracer analyte molecule being attached to a respective magnetic bead;
- a magnet arrangement comprising:
- a first electromagnetic magnet (M1) configured to be powered to attract the plurality of tracer analyte molecules to the collection surface; and
- a second electromagnetic magnet (M2) configured to be powered to attract the plurality of tracer analyte molecules to the capture surface;
- a power supply configured to supply power to the magnet arrangement; and
- a controller configured to control the power supply to:
- depower the first electromagnetic magnet and the second electromagnetic magnet to release the tracer analyte molecules from the collection surface to allow for binding of the plurality of tracer analyte molecules to the receptors on the capture surface after an incubation time; and
- power, after the incubation time, the first electromagnetic magnet to apply a magnetic field of a defined field strength;
- a detector configured to detect an amount of tracer analyte molecules on one of the capture surface and the collection surface; and
wherein the measurement unit is configured to correlate the detected amount of tracer analyte molecules on one of the capture surface and the collection surface with the quality of the capture surface.

13. System according to any one of the preceding claims, further comprising:
- a user interface configured to notify a user of the measured value.

14. A method (200) for lifetime prediction or lifetime detection of an analyte sensor, the method comprising:
- providing (210) an analyte sensor that comprises:
- an analyte sensor matrix having a capture surface with receptors being immobilized thereon for reversibly binding analyte molecules in the fluid sample; and
- a regeneration assembly configured to regenerate the capture surface such that the bound analyte molecules are released from the capture surface; and
- performing (220) measurements, by a measurement unit, to provide a measured value correlated with a quality of the capture surface, wherein the measured value allows for determining when the analyte sensor matrix has degraded and/or for predicting when the analyte sensor matrix will degrade.

15. A program element for lifetime prediction or lifetime detection of an analyte sensor, which program element, when being executed by a processor, is adapted to carry out the method according to claim 14.
